# EUROPEAN PATENT APPLICATION

(11) **EP 4 574 021 A1**
(43) Date of publication of application: **25.06.2025**
(21) Application number: 23218467.1
(22) Date of filing: 20.12.2023
(51) Int. Cl.: A61B 5/00, A61B 5/055, A61B 5/0295, A61B 5/0507, A61B 5/11

(54) **PROVIDING A CARDIAC SIGNAL**

(71) Applicant: Koninklijke Philips N.V., 5656 AG Eindhoven (NL)
(72) Inventor: SENEGAS, Julien Thomas, Eindhoven (NL); WUELBERN, Jan Hendrik, Eindhoven (NL); FRERKING, Lena Christina, 5656AG Eindhoven (NL); KOEHLER, Thomas, Eindhoven (NL); SCHMITT, Holger, Eindhoven (NL); WEISS, Steffen, Eindhoven (NL)
(74) Representative: Philips Intellectual Property & Standards

(57) **Abstract**

A system (100) for providing a cardiac signal (S_{Cardiac}), is provided. The system includes one or more processors (110) configured to: receive medical image data (120) representing a beating of a heart of a subject; receive initial surrogate cardiac signal data (130) comprising an initial surrogate cardiac signal (S_{PPG}) representing the beating of the heart of the subject, the initial surrogate cardiac signal data (130) being generated contemporaneously with the medical image data (120); analyze the medical image data (120) to provide a reference cardiac signal (S_{Image}) representing the beating of the heart of the subject; determine a correspondence (T_{D}) between the reference cardiac signal (S_{Image}) and the initial surrogate cardiac signal (S_{PPG}); receive subsequent surrogate cardiac signal data (130') comprising a subsequent surrogate cardiac signal (S_{PPG}') representing the beating of the heart of the subject; generate the cardiac signal (S_{Cardiac}) based on the subsequent surrogate cardiac signal (S_{PPG}') and the correspondence (T_{D}); and output the cardiac signal (S_{Cardiac}).

## Description

### TECHNICAL FIELD

The present disclosure relates to providing a cardiac signal. A system, a computer-implemented method, a computer program product, and a medical imaging system that includes the system, are disclosed.

### BACKGROUND

Cardiac signals are routinely acquired by physicians. The cardiac signals may be acquired for various purposes. For instance, cardiac signals are acquired for the purpose of investigating the functioning of the heart. In such situations the cardiac signals may provide information such as the heart rate and the heart rate variability, which indicate the health of a subject. Cardiac signals are also acquired for other purposes, such as to select medical image data for use in image reconstruction via cardiac gating, and to trigger the delivery of radiation therapy to a subject via cardiac gating.

Cardiac gating is used for image reconstruction purposes with imaging modalities such as computed tomography "CT", magnetic resonance imaging "MRI", and positron emission tomography "PET". Cardiac gating-based image reconstruction may be performed in a so-called "prospective gating" mode, or in a so-called "retrospective gating" mode. In the prospective gating mode, a cardiac signal is used to perform the acquisition of medical image data only for specific parts in the cardiac cycle, usually by starting (i.e. triggering) the acquisition at a specific point in the cardiac cycle followed by data acquisition for a constant period of time. The medical image data from this specific parts is then reconstructed to provide a medical image corresponding to a specific phase of the cardiac cycle. In the retrospective gating mode, medical image data is acquired throughout (or over a large part of) the cardiac cycle and a cardiac signal is used to apply timestamps to the medical image data the represent different points in the cardiac cycle. The timestamps are then used to select data for reconstruction so as to provide a medical image corresponding to the specific phase of the cardiac cycle. Usually, the goal of cardiac gating is to acquire data during a phase of the cardiac cycle where the cardiac motion is small. By reconstructing medical images from data that is acquired only at these specific phases of the cardiac cycle, it is ensured that the anatomy is in a similar position. This reduces image artifacts such as cardiac motion-induced blurring. The use of cardiac gating to trigger the delivery of radiation therapy to a subject likewise reduces the impact of cardiac motion. Here, the intensity of a radiation therapy beam is modulated based on a cardiac signal so as to ensure that the heart, and consequently the anatomy of a subject, is in a known position in the periods in which the radiation is delivered to the anatomy.

Cardiac signals are often acquired by placing electrodes on the skin of a subject and acquiring a so-called electrocardiogram, or "ECG". The electrodes detect electrical signals that are generated as a result of cardiac muscle depolarization and re-polarization during the cardiac cycle. An ECG may be considered to represent the gold standard measurement of cardiac signals due to its direct correspondence with the activity of the cardiac muscle. However, there are drawbacks associated with acquiring cardiac signals in the form of an ECG. For instance, attaching the electrodes to the skin in the required locations is time-consuming, and also inconvenient for a subject. The electrodes, and also their leads, may also obstruct features in medical images.

Other techniques for acquiring cardiac signals include the use of photoplethysmography to acquire a photoplethysmogram "PPG", and the use of ballistocardiography to acquire a ballistocardiograph "BCG". A PPG is an optically acquired plethysmogram that represents blood volume changes in a tissue bed. A PPG is typically acquired using a pulse oximeter which measures changes in optical absorption of the skin. A PPG may be acquired by measuring the optical absorption on skin regions such as the finger, the ear, nasal septum, and the forehead. A PPG, and also a BCG, may be acquired using contact with the body, and also using contact-less techniques. A PPG, and also a BCG, are both easier to acquire than an ECG. However, both a PPG, and also a BCG, have a weaker relationship with the activity of the cardiac muscle. This typically limits their use to the provision of information such as heart rate, and heart rate variability.

Consequently, there is a need to improve the accuracy of surrogate cardiac signals, such as those acquired via photoplethysmography, and via ballistocardiography, in order to take advantage of their benefits.

### SUMMARY

According to one aspect of the present disclosure, a system for providing a cardiac signal, is provided. The system comprises one or more processors configured to:
receive medical image data representing a beating of a heart of a subject;
receive initial surrogate cardiac signal data comprising an initial surrogate cardiac signal representing the beating of the heart of the subject, the initial surrogate cardiac signal data being generated contemporaneously with the medical image data;
analyze the medical image data to provide a reference cardiac signal representing the beating of the heart of the subject;
determine a correspondence between the reference cardiac signal and the initial surrogate cardiac signal;
receive subsequent surrogate cardiac signal data comprising a subsequent surrogate cardiac signal representing the beating of the heart of the subject;
generate the cardiac signal based on the subsequent surrogate cardiac signal and the correspondence; and
output the cardiac signal.

In the above system, medical image data is analyzed in order to provide a reference cardiac signal representing the beating of the heart of the subject. Since the reference cardiac signal is obtained from medical image data, it provides a reliable reference for the beating of the heart. The correspondence between the reference cardiac signal and an initial surrogate cardiac signal is then determined. The correspondence is then used to generate the cardiac signal from a subsequent surrogate cardiac signal. The correspondence may be a delay between the reference cardiac signal and the initial surrogate cardiac signal, for example. Thus, by using the correspondence to generate the cardiac signal, the delay may be accounted-for, and a more accurate cardiac signal is provided.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for providing a cardiac signal S_{Cardiac}, in accordance with some aspects of the present disclosure.
Fig. 2 is a flowchart illustrating an example of a computer-implemented method of providing a cardiac signal, in accordance with some aspects of the present disclosure.
Fig. 3 is a schematic diagram illustrating an example of various elements of a computer-implemented method of providing a cardiac signal S_{Cardiac}, in accordance with some aspects of the present disclosure.

### DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, and in a computer program product, and in a medical imaging system, and in a radiation therapy delivery system, in a corresponding manner.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the systems disclosed herein may be implemented using artificial intelligence techniques. Suitable techniques may include machine learning techniques, as well as deep learning techniques such as neural networks. For instance, one or more neural networks, may be trained in a supervised, or in some cases unsupervised, manner, to implement the operations that are performed by the one or more processors.

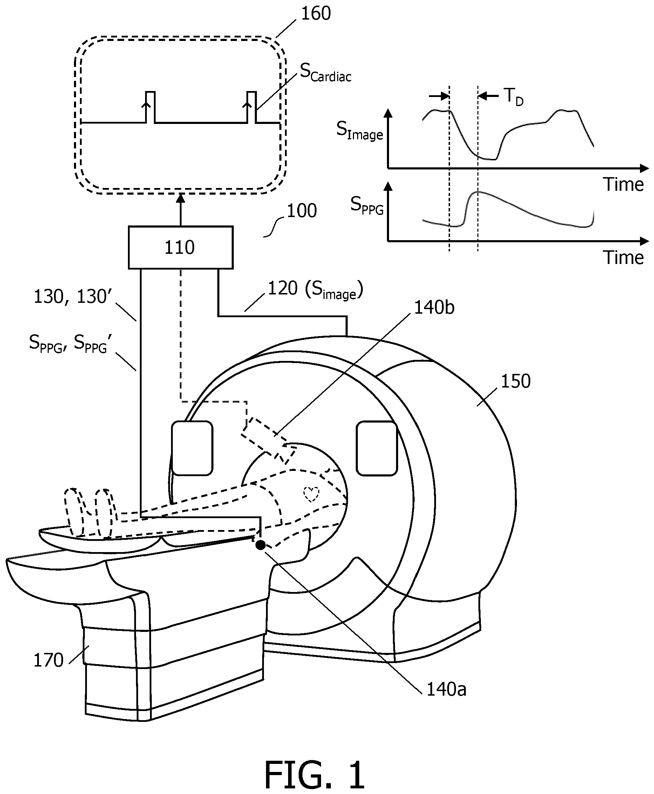

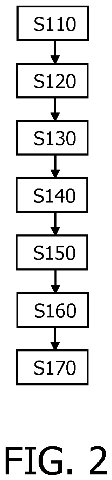

As mentioned above, there is a need to improve the accuracy of surrogate cardiac signals, such as those acquired via photoplethysmography, and via ballistocardiography. Fig. 1 is a schematic diagram illustrating an example of a system 100 for providing a cardiac signal S_{Cardiac}, in accordance with some aspects of the present disclosure. Fig. 2 is a flowchart illustrating an example of a computer-implemented method of providing a cardiac signal, in accordance with some aspects of the present disclosure. It is noted that operations that are described as being performed by the one or more processors 110 of the system 100 illustrated in Fig. 1, may also be performed in the method illustrated in Fig. 2. Likewise, operations that are described in relation to the method described with reference to Fig. 2 may also be performed by the one or more processors 110 of the system 100 illustrated in Fig. 1. With reference to Fig. 1, and Fig. 2, the system 100 for providing a cardiac signal S_{Cardiac}, comprises one or more processors 110 configured to:
receive S110 medical image data 120 representing a beating of a heart of a subject;
receive S120 initial surrogate cardiac signal data 130 comprising an initial surrogate cardiac signal S_{PPG} representing the beating of the heart of the subject, the initial surrogate cardiac signal data 130 being generated contemporaneously with the medical image data 120;
analyze S130 the medical image data 120 to provide a reference cardiac signal S_{Image} representing the beating of the heart of the subject;
determine S140 a correspondence T_{D} between the reference cardiac signal S_{Image} and the initial surrogate cardiac signal S_{PPG};
receive S150 subsequent surrogate cardiac signal data 130' comprising a subsequent surrogate cardiac signal S_{PPG}' representing the beating of the heart of the subject;
generate S160 the cardiac signal S_{Cardiac} based on the subsequent surrogate cardiac signal S_{PPG}' and the correspondence T_{D}; and
output S170 the cardiac signal S_{Cardiac}.

In the above system, medical image data is analyzed in order to provide a reference cardiac signal representing the beating of the heart of the subject. Since the reference cardiac signal is obtained from medical image data, it provides a reliable reference for the beating of the heart. The correspondence between the reference cardiac signal and an initial surrogate cardiac signal is then determined. The correspondence is then used to generate the cardiac signal from a subsequent surrogate cardiac signal. The correspondence may be a delay between the reference cardiac signal and the initial surrogate cardiac signal, for example. Thus, by using the correspondence to generate the cardiac signal, the delay may be accounted-for, and a more accurate cardiac signal is provided.

The operations that are performed by the one or more processors 110 of the system 100 are described in more detail below.

Referring initially to the operation S 110 illustrated in Fig. 2; in this operation, medical image data 120 is received. The medical image data 120 represents a beating of a heart of a subject.

The medical image data 120 may be generated by various types of medical imaging systems, including two-dimensional "2D" imaging systems, and three-dimensional "3D", or "volumetric", imaging systems. Examples of 2D medical imaging systems include (spectral) X-ray projection imaging systems, and 2D ultrasound imaging systems. Examples of volumetric imaging systems include MRI systems, (spectral) computed tomography "CT" imaging systems, positron emission tomography "PET" imaging systems, single photon emission computed tomography "SPECT" imaging systems, 3D ultrasound imaging systems, and so forth. In the example illustrated in Fig. 1, the medical image data 120 is generated by an MRI system 150.

The medical image data 120 that is received in the operation S 110 represents a beating of a heart of a subject. In this regard, the medical image data 120 may represent at least a portion of the heart. For instance, the medical image data 120 may represent a chamber of the heart, such as the left- or the right-ventricle, or a valve of the heart, and so forth. Portions of the heart such as these represent the beating of a heart via their pulsation in accordance with the cardiac cycle. For instance, the left ventricle contracts and relaxes as it pumps blood during the cardiac cycle. Likewise, valves within the heart such as the pulmonary valve, the tricuspid valve, and so forth, open and close during the cardiac cycle. The medical image data 120 may alternatively represent the beating of a heart in a different manner. For instance, portions of the vasculature that are associated with the heart, such as the aorta, and which are not part of the heart, also pulsate in accordance with the cardiac cycle. Thus, the medical image data 120 may alternatively represent a portion of the vasculature associated with the with the heart, such as at least a portion of the aorta, and which also indicate the beating of the heart via their pulsation in accordance with the cardiac cycle.

In some examples, the medical image data 120 is so-called raw data, i.e. data that has not yet been reconstructed into an image. In other examples, the medical image data 120 is reconstructed image data, i.e. data that has already been reconstructed to provide an image. Raw data generated by an MRI system may be referred-to as k-space data. Raw data that is generated by an MRI system may be referred-to as projection CT data. Thus, the medical image data 120 may include a temporal sequence of images that represent the beating of a heart of a subject. Alternatively, the medical image data 120 may include a temporal sequence of raw datasets that represent the beating of the heart of the subject.

In one example, the medical image data 120 that is received in the operation S110 represents a diagnostic imaging scan of a subject. A diagnostic imaging scan includes sufficient information to perform a diagnosis of a subject. However, in some situations, a diagnostic imaging scan may not be available. It may also be impermissible to perform a dedicated diagnostic imaging scan in order to provide the medical image data 120 due to the additional burden on workflow, or due to the increase X-ray dose to a subject. In another example, the medical image data 120 represents a surview scan of the subject, or scan of an injected contrast agent bolus in the subject. Such scans are typically acquired as part of the conventional workflow during medical imaging procedures, and they typically include sufficient information to represent the beating of a heart of a subject. Thus, the use of such scans to provide the medical image data 120 simplifies workflow and/or reduces X-ray dose to a subject.

A surview scan is also known as a localizer radiograph, or a scanogram, or a scout scan. A surview scan is typically performed as part of the conventional workflow prior to a diagnostic imaging scan. A surview scan is also typically performed at a relatively lower dose than a diagnostic imaging scan. A surview typically includes sufficient information to represent the beating of a heart of a subject because it typically captures the pulsation of the heart, either by capturing a portion of the heart, or by capturing a portion of the vasculature associated with the heart, such as the aorta, and which likewise pulsates in accordance with the cardiac cycle. The surview scan may be a low-dose helical CT surview scan for example. A low-dose helical CT surview is often performed in order to localize the heart prior to performing a diagnostic imaging scan of the heart during a CT imaging procedure. Similarly, a scan of an injected contrast agent bolus in the subject is also often performed as part of the conventional workflow prior to a diagnostic imaging scan. The contrast agent may have been injected in order to assess blood flow in the vasculature, or to navigate an interventional device within the vasculature. A scan of an injected contrast agent bolus typically includes sufficient information to represent the beating of a heart of a subject because it also typically captures a portion of the heart, or a portion of the vasculature associated with the heart, such as the aorta, and which likewise pulsates in accordance with the cardiac cycle.

The medical image data 120 that is received in the operation S 110 may be received via any form of data communication, including via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

Referring now to the operation S 120 illustrated in Fig. 2; in this operation, initial surrogate cardiac signal data 130 is received. The initial surrogate cardiac signal data 130 comprises an initial surrogate cardiac signal S_{PPG} representing the beating of the heart of the subject. The initial surrogate cardiac signal data 130 is generated contemporaneously with the medical image data 120.

The initial surrogate cardiac signal data 130 may be generated by various types of sensors, including for example a photoplethysmography, PPG, sensor 140a, 140b, or by a ballistocardiographic, BCG, sensor, or by a pulse oximetry sensor, or by a pressure sensor, or by a radar sensor. Such sensors generate surrogate cardiac signals, i.e. cardiac signals represent indirect measurements of the activity of the cardiac muscle. The initial surrogate cardiac signal data 130 may be generated by a sensor that contacts the subject, or alternatively by a so-called "contact-less" sensor that measures a cardiac signal without contacting the subject.

A PPG sensor measures optical absorption on a skin region such as a finger, an ear, the nasal septum, and the forehead. The optical absorption in such regions varies in accordance with blood flow, and the blood flow varies during the cardiac cycle. In so doing, a PPG sensor provides a surrogate cardiac signal that represents the beating of the heart of the subject. A PPG sensor may contact the skin. For instance the PPG sensor may be provided in the form of a finger clip, an ear clip, and so forth. A contact-less PPG sensor may alternatively be provided in the form of a camera that measures the optical absorption on such skin regions without contacting the subject. The camera typically measures differences in the intensity of the optical absorption of the skin region arising from deoxygenated, and oxygenated haemoglobin, over different color channels.

A BCG sensor provides a measurement of a subject's motion arising from the ejection of the blood during the cardiac cycle. In so doing, a BCG sensor provides a surrogate cardiac signal that represents the beating of the heart of the subject. A BCG sensor may be provided by various types of motion sensors. The motion sensor may measure a position, or a velocity, or an acceleration of the subject. The BCG sensor may be mechanically coupled to the subject, or alternatively it may be mechanically coupled to a subject support, such as a patient bed.

A pulse oximetry sensor measures blood oxygen levels and is typically provided in the form of a clip-type device for attachment to a finger, or an earlobe, or a foot. A pulse oximetry sensor measures blood oxygen levels via the optical absorption of such regions. The optical absorption is dependent upon the blood flow, which varies during the cardiac cycle. Consequently, a pulse oximetry sensor provides a surrogate cardiac signal that represents the beating of the heart of the subject.

Similarly, a pressure sensor may be mechanically coupled to a subject, for instance to a limb of a subject. The pressure sensor measures the blood pressure in the subject. The blood pressure varies during the cardiac cycle. Thus, a pressure sensor may provide a surrogate cardiac signal that represents the beating of the heart of the subject. A radar sensor may be used to measure a subject's motion arising from the ejection of the blood during the cardiac cycle in a similar manner a BCG sensor. Thus, a radar sensor may provide a surrogate cardiac signal that represents the beating of the heart of the subject.

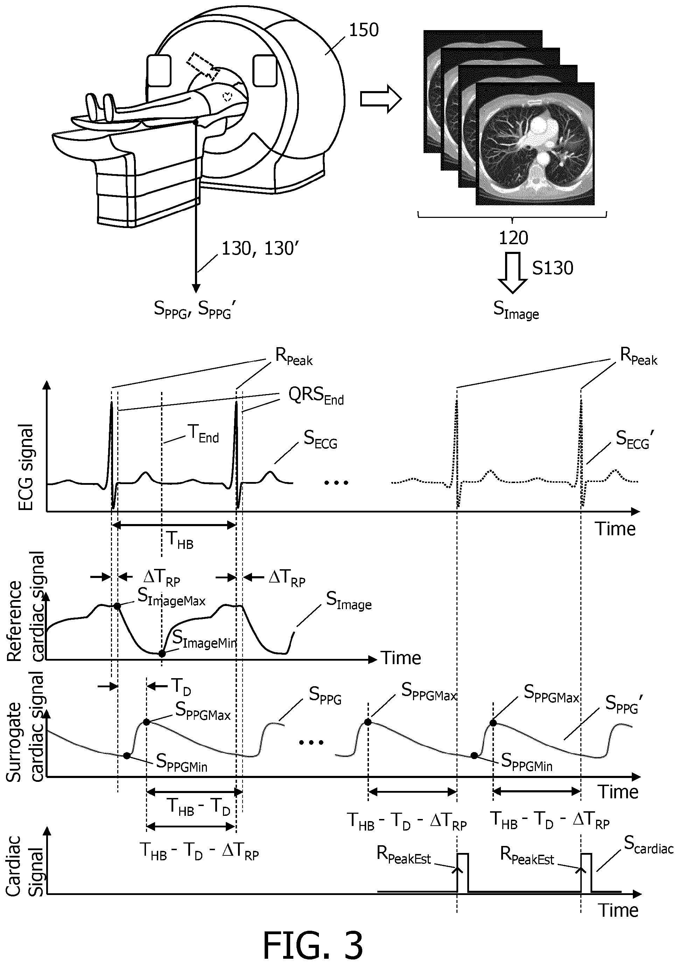

An example of an initial surrogate cardiac signal S_{PPG} representing the beating of the heart of the subject, is shown in the graph labelled "Surrogate cardiac signal" in Fig. 3. Fig. 3 is a schematic diagram illustrating an example of various elements of a computer-implemented method of providing a cardiac signal S_{Cardiac}, in accordance with some aspects of the present disclosure. In the example illustrated in Fig. 3, the initial surrogate cardiac signal S_{PPG} is generated by a PPG sensor. The PPG sensor is in contact with a finger of a subject, as illustrated in the upper portion of Fig. 3. In this example, the value of the initial surrogate cardiac signal S_{PPG} corresponds to the optical absorption within a finger. As mentioned above, the optical absorption within a finger varies in accordance with the blood flow in the finger. The blood flow varies during the cardiac cycle. A maximum value of the initial surrogate cardiac signal S_{PPGMax} corresponds to an instant of maximum optical absorption in the finger, i.e. the instant of maximum blood volume in the finger, and a minimum value of the initial surrogate cardiac signal S_{PPGMin} corresponds to an instant of minimum optical absorption in the finger, i.e. the instant of minimum blood volume in the finger.

The initial surrogate cardiac signal data 130 that is received in the operation S 120 is generated contemporaneously with the medical image data 120. Consequently, the initial surrogate cardiac signal data 130 corresponds, time-wise, to the medical image data 120. The initial surrogate cardiac signal data 130, and the medical image data 120, may be acquired over a period of time, such as over one or more cardiac cycles.

Referring now to the operation S130 illustrated in Fig. 2; in this operation, the medical image data 120 is analyzed to provide a reference cardiac signal S_{Image} representing the beating of the heart of the subject.

Various techniques may be used to provide the reference cardiac signal S_{Image} in the operation S130. In one example, the medical image data 120 comprises a temporal sequence of images including at least a portion of the heart and/or at least a portion of the aorta. In this example, the analyzing S 130 the medical image data 120 comprises:
reconstructing at least a portion of the medical image data 120 to provide a temporal sequence of images representing the at least a portion of the heart and/or the at least a portion of the aorta; and
extracting the reference cardiac signal S_{Image} representing the beating of the heart of the subject, from the temporal sequence of images.

As mentioned above, the at least a portion of the heart, and likewise the at least a portion of the aorta, pulsate in accordance with the cardiac cycle, and so these regions may be used to provide a cardiac signal representing the beating of the heart. In one example, the medical image data is reconstructed to provide complete images, e.g. volumetric images, or image slices, that represent the complete field of view imaged by the medical image data. In another example, the medical image data is reconstructed to provide partial images that represent only a portion of the complete field of view imaged by the medical image data and which includes the at least a portion of the heart and/or the at least a portion of the aorta. The reconstruction may be performed using known techniques. The operation of extracting the reference cardiac signal S_{Image} may be performed using known image processing techniques. For instance, in one example, the operation of extracting the reference cardiac signal S_{Image} comprises:
evaluating an image intensity within a region of interest in the images; and
generating the reference cardiac signal S_{Image} based on the evaluated image intensities in the images.

In one example, the region of interest corresponds to a cardiac chamber. The cardiac chamber may be the left ventricle, or the right ventricle, or another cardiac chamber. The size of the left ventricle provides a reliable cardiac signal S_{Image} because its size defines known points in the cardiac cycle.

An example of the reference cardiac signal S_{Image} representing the beating of the heart of the subject that results from the operation S 130, is shown in the graph labelled "Reference cardiac signal" in Fig. 3. In this example, the reference cardiac signal S_{Image} represents the volume of the left ventricle of the heart. The reference cardiac signal S_{Image} is obtained by reconstructing a temporal sequence of CT images that include the left ventricle, and evaluating the image intensities within the images in a region of interest that corresponds to the left ventricle. For reference purposes, the graph labelled "ECG signal", S_{ECG}, is also shown in Fig. 3. This graph represents a contemporaneously-acquired ECG signal that would have been acquired by monitoring the heart using electrodes. The ECG signal, S_{ECG}, is also labelled with various reference points in the cardiac cycle, T_{HB}, such as the so-called "R-peak", labelled R_{Peak}, the end of the so-called "QRS complex", labelled QRS_{End}, and the end of the so-called T-wave, labelled T_{End}.

As may be seen in the graph labelled "Reference cardiac signal" in Fig. 3, the size of the left ventricle, represented in this graph by the Reference cardiac signal, S_{Image}, has known relationship with the cardiac cycle, T_{HB}. This relationship is often referred-to as the Wiggers diagram. The maximum value of the Reference cardiac signal, S_{ImageMax}, i.e. the maximum size of the left ventricle, coincides with the end of the so-called QRS complex", labelled QRS_{End} in the cardiac cycle, T_{HB}. The minimum value of the Reference cardiac signal, S_{ImageMin}, i.e. the minimum size of the left ventricle, coincides with the end of the so-called T-wave, labelled T_{End} in the cardiac cycle, T_{HB}. The low variation of the length of the QRS complex in patients, typically 80 milliseconds to 100 milliseconds, allows the estimation of the so-called R-peak, labelled R_{Peak} in the cardiac cycle, T_{HB}, based on its relationship with the point of maximum left ventricular volume, i.e. S_{ImageMax}, in the Reference cardiac signal, S_{Image}. Reference points such as these are important because they represent defined points in the cardiac cycle, T_{HB}. For instance, the R-peak in an ECG cardiac signal is commonly used as a trigger point in cardiac gating applications. Thus, the known relationship between the Reference cardiac signal S_{Image} and the cardiac cycle, T_{HB}, can be used to predict the occurrence of reference points in the cardiac cycle T_{HB}, such as QRS_{End}, T_{End}, and R_{Peak}, from a measurement of the Reference cardiac signal S_{Image}.

An example of the analyzing operation S130 is described below in which the medical image data 120 represents the left ventricle. The size of the left ventricle serves as a good region of interest to evaluate in the medical image data because it represents the largest myocardial mass, and also because of its known relationship with the cardiac cycle, T_{HB}, as described above. In this example, the medical image data 120 may be generated from a surview scan. The medical image data 120 is reconstructed to provide a temporal series of partial images representing the left ventricle. The images may be partial in the sense that they may represent only part of the field of view of view imaged by the medical imaging system. Image processing techniques, or a neural network, may be used to identify the left ventricle in the partial images, and to calculate its size. The size of the left ventricle may be calculated based on its volume, or its area, or another dimension in the partial images. The size of the left ventricle is then used as the reference cardiac signal S_{Image}. Instead of calculating the size of the left ventricle, an image intensity within a region of interest in the partial images may be used as the reference cardiac signal S_{Image}. The region of interest may correspond to a cardiac chamber, such as the left ventricle, or it may correspond to another portion of the vasculature associated with the heart, such as the aorta. The image intensities within such regions of interest also represent the beating of the heart because they typically capture a portion of the heart, or a portion of the vasculature associated with the heart, such as the aorta, and which pulsates in accordance with the cardiac cycle. Having obtained the reference cardiac signal S_{Image}, the time points of minimal and maximal cardiac contraction, i.e. the time points S_{ImageMax} and S_{ImageMin} in the reference cardiac signal S_{Image} may be determined.

In this example, the medical image data 120 from the surview scan may include some data redundancy. For instance, multiple low-resolution images, or multiple partial images, each corresponding to a different point in the cardiac cycle, or a different phase of the cardiac cycle, may be reconstructed from the medical image data. In the example of the medical image data 120 being provided by a CT imaging system, the surview data may be provided by a so-called low-dose helical scan. The raw data may be binned into time intervals, each corresponding to a phase of the cardiac cycle, each with a duration of 100 milliseconds, for example, to provide a temporal series of partial reconstructed images representing the contraction of the heart over a complete cardiac cycle. Image processing techniques such as those described in a document by Lessick, J., et al., "Automatic determination of differential coronary artery motion minima for cardiac computed tomography optimal phase selection", Acad. Radiol. 2015 Jun;22(6):697-703, may be applied to the image to identify different phases of the cardiac cycle (e.g. diastole and systole). In the example of the medical image data 120 being provided by an MRI system, the surview data the medical image data may be provided in subsets interleaved in k-space. Lowly-resolved images may then be reconstructed from these subsets. A measure of the left ventricular volume over time may then be derived by applying known image processing techniques, or using a neural network, to the images.

Referring now to the operation S140 illustrated in Fig. 2; in this operation, a correspondence T_{D} between the reference cardiac signal S_{Image} and the initial surrogate cardiac signal S_{PPG}, is determined. This correspondence is determined so that it can be used to provide an association between a subsequently-acquired surrogate cardiac signal S_{PPG}', and the cardiac cycle, T_{HB}.

The correspondence may be a time difference, or a phase difference, between the reference cardiac signal S_{Image} and the initial surrogate cardiac signal S_{PPG}. In one example, the correspondence is a time delay, or a phase delay, between the reference cardiac signal S_{Image} and the initial surrogate cardiac signal S_{PPG}. The correspondence may be a time difference, or a phase difference, between a reference point in the reference cardiac signal S_{Image}, such as the reference point S_{ImageMax} or the reference point S_{ImageMin}, and a trigger point in the initial surrogate cardiac signal S_{PPG}, such as the trigger point S_{PPGMax}, or the trigger point S_{PPGMin}. The trigger point may alternatively be another trigger point in the initial surrogate cardiac signal S_{PPG}, such as the maximum value of a derivative of the initial surrogate cardiac signal S_{PPG}, or a minimum value of a derivative of the initial surrogate cardiac signal S_{PPG}, or a zero crossing value of a Hilbert transform of the initial surrogate cardiac signal S_{PPG}. Such reference points, and trigger points, may be used because they are readily-identifiable in these signals. The value of the correspondence may be determined in the operation S140 using known signal processing techniques. In one example, the value of the correspondence may be determined by comparing a time, or a phase, of a reference point in the reference cardiac signal S_{Image} with a time, or a phase, of a trigger points in the initial surrogate cardiac signal S_{PPG}. In another example, the value of the correspondence may be determined by evaluating a correlation between the reference cardiac signal S_{Image} and the initial surrogate cardiac signal S_{PPG}.

Referring now to the operation S150 illustrated in Fig. 2; in this operation subsequent surrogate cardiac signal data 130' is received. The subsequent surrogate cardiac signal data 130' comprises a subsequent surrogate cardiac signal S_{PPG}' representing the beating of the heart of the subject.

The subsequent surrogate cardiac signal data 130' is generated by the same sensor as the initial surrogate cardiac signal data 130, and is simply generated at a later point in time than the initial surrogate cardiac signal data 130. The later point in time may correspond to a period within a diagnostic imaging scan, or a period within a radiation therapy session, for example. An example of subsequent surrogate cardiac signal data 130' is illustrated on the right-hand side of Fig. 3, i.e. as the subsequent surrogate cardiac signal S_{PPG}'. One or more trigger points may be established in the subsequent surrogate cardiac signal S_{PPG}'. These trigger point(s) correspond to the trigger points mentioned above. For instance, the one or more trigger points may correspond to a maximum value S_{PPGMax} of the subsequent surrogate cardiac signal S_{PPG}', or a minimum value S_{PPGMin} of the subsequent surrogate cardiac signal S_{PPG}', or a maximum value of a derivative of the subsequent surrogate cardiac signal S_{PPG}', or a minimum value of a derivative of the subsequent surrogate cardiac signal S_{PPG}', or a zero crossing value of a Hilbert transform of the subsequent surrogate cardiac signal S_{PPG}'.

Referring now to the operation S160 illustrated in Fig. 2; in this operation, the cardiac signal S_{Cardiac} is generated based on the subsequent surrogate cardiac signal S_{PPG}' and the correspondence T_{D}.

In one example, the cardiac signal S_{Cardiac} is generated by using the correspondence T_{D} to delay the subsequent surrogate cardiac signal S_{PPG}'. By way of an example, the correspondence T_{D} may be a time delay between the reference point S_{ImageMax} in the reference cardiac signal S_{Image}, and the trigger point S_{PPGMax} in the initial surrogate cardiac signal S_{PPG}. This example is illustrated in Fig. 3. Delaying the subsequent surrogate cardiac signal S_{PPG}' by a duration T_{HB} - T_{D}, wherein T_{HB} is the period of the cardiac cycle, results in a delayed version of the subsequent surrogate cardiac signal S_{PPG}', i.e. a cardiac signal S_{Cardiac}, in which the trigger point S_{PPGMax} in the subsequent surrogate cardiac signal S_{PPG} is synchronized with the expected time of the reference point S_{ImageMax} in the reference cardiac signal S_{Image}. More generally, in this example, the correspondence comprises a time delay T_{D}, or a phase delay, between a reference point in the reference cardiac signal S_{Image}, and a trigger point in the initial surrogate cardiac signal S_{PPG}, and the cardiac signal S_{Cardiac} is generated by using the correspondence T_{D} to delay the subsequent surrogate cardiac signal S_{PPG}' by a duration T_{HB} - T_{D}, wherein T_{HB} is the period of the cardiac cycle. This results in the trigger point in the subsequent surrogate cardiac signal S_{PPG} being synchronized with the expected time or phase of the reference point in the reference cardiac signal. This is a useful cardiac signal because it includes an indication, via the delayed trigger point S_{PPGMax}, of the expected time of a known point in the cardiac cycle, i.e. the expected time of the reference point S_{ImageMax} in the reference cardiac signal, which as mentioned above, corresponds to the end of the QRS complex in the cardiac cycle, i.e. the point QRS_{End} in the signal S_{ECG} in Fig. 3.

It is noted that the other trigger points mentioned above, such as S_{PPGMin}, the maximum value of a derivative of the subsequent surrogate cardiac signal S_{PPG}', and so forth, and also the other reference points mentioned above, such as S_{ImageMin} may be used in a similar manner to provide a cardiac signal. For instance, delaying the subsequent surrogate cardiac signal S_{PPG}' so as to provide the subsequent surrogate cardiac signal S_{PPG}' with an indication of the expected time of the reference point S_{ImageMin} in the reference cardiac signal, is also a useful cardiac signal because the reference point S_{ImageMin} corresponds to the end of the T-wave in the cardiac cycle, i.e. the point T_{End} in the signal S_{ECG} in Fig. 3.

In another example, instead of simply delaying the subsequent surrogate cardiac signal S_{PPG}' to provide a cardiac signal that has an indication (i.e. the trigger point S_{PPGMax}) of the expected time of the reference point S_{ImageMax}, S_{ImageMin}, in the reference cardiac signal S_{Image}, the indication may be provided in a different manner. For instance, a cardiac signal may be provided which includes a leading edge, and wherein the time, or the phase, of the leading edge is coincident with the expected time of the reference point S_{ImageMax} , S_{ImageMin} in the reference cardiac signal S_{Image}. Thus, in this example, the leading edge serves as the indication of the expected time of the reference point S_{ImageMax}, S_{ImageMin} in the reference cardiac signal S_{Image}.

In another example, a known time or phase relationship ΔT_{RP} between the reference cardiac signal S_{Image} and the reference point R_{Peak}, QRS_{End}, T_{End} in the cardiac cycle, T_{HB}, is used to generate a cardiac signal S_{Cardiac} that includes an indication of an expected time R_{PeakEst}, or an expected phase, of a reference point R_{Peak}, QRS_{End}, T_{End} in a cardiac cycle T_{HB} of the heart.

In this example, the operation of generating S 160 the cardiac signal S_{Cardiac} comprises generating an indication of an expected time R_{PeakEst}, or an expected phase, of a reference point R_{Peak}, QRS_{End}, T_{End} in a cardiac cycle T_{HB} of the heart, and wherein the indication is generated based on the subsequent surrogate cardiac signal S_{PPG}', and the correspondence T_{D}, and based further on a known time or phase relationship ΔT_{RP} between the reference cardiac signal S_{Image} and the reference point R_{Peak}, QRS_{End}, T_{End} in the cardiac cycle T_{HB}.

This example is illustrated in reference Fig. 3, for the situation in which the cardiac signal S_{Cardiac} (illustrated at the bottom of Fig. 3) includes an indication (via the leading edge of the pulse in the cardiac signal S_{Cardiac} of the expected time R_{PeakEst} of the reference point R_{Peak} in the cardiac cycle T_{HB} of the heart. The expected time R_{PeakEst} of the reference point R_{Peak} in the cardiac cycle T_{HB} of the heart is illustrated via the dotted trace in the graph labelled "ECG signal". The reference point R_{Peak} is referred-to as the R-peak, and it commonly serves as a trigger point for cardiac gating applications. The R-peak in the cardiac cycle, T_{HB}, is known a high degree of certainty to occur 80 milliseconds to 100 milliseconds prior to the time of maximum volume of the left ventricle. Thus, in this example, the time delay ΔT_{RP} = 80 to 100 milliseconds, provides a known time relationship between the reference cardiac signal S_{Image} and the reference point R_{Peak} in the cardiac cycle T_{HB}. In the example illustrated in Fig. 3, the cardiac signal S_{Cardiac} is generated by providing a pulse with a leading edge that occurs at a time *T_{HB}* - *T_{D}* - *ΔT_{RP}* after the trigger point S_{PPGMax}.

Instead of providing an indication of the expected time of the R-peak in the cardiac cycle, the expected times of other reference points in the cardiac cycle of the heart, may be indicated in this example. For instance, the expected time of the reference point QRS_{End}, i.e. the end of the QRS complex, or the reference point T_{End}, i.e. the end of the T-wave, may be indicated. For these reference points, there is also a known time or phase relationship between the reference cardiac signal S_{Image} and the reference point in the cardiac cycle T_{HB}. For example, the reference point QRS_{End}, coincides with the time of maximum volume of the left ventricle, i.e. the time of the maximum value of the Reference cardiac signal, S_{ImageMax}. Likewise, the reference point T_{End}, coincides with the time of minimum volume of the left ventricle, i.e. the time of the minimum value of the Reference cardiac signal, S_{ImageMin}. The ability to provide an indication, via the cardiac signal S_{Cardiac}, of such reference points in the cardiac cycle improves the accuracy of the cardiac signal and facilitate its use in applications such as cardiac gating.

In another example, a trigger point is established in the subsequent surrogate cardiac signal S_{PPG}', and this is used in combination with the known time or phase relationship ΔT_{RP} described above, to generate a cardiac signal S_{Cardiac}. In this example, the operation of determining S 140 a correspondence T_{D} between the reference cardiac signal S_{Image} and the initial surrogate cardiac signal S_{PPG}, comprises:
establishing a trigger point S_{PPGMax}, S_{PPGMin} in the subsequent surrogate cardiac signal S_{PPG}'; and
calculating a time difference, or a phase difference, between a reference point S_{ImageMax} in the reference cardiac signal S_{Image} and the trigger point S_{PPGMax}, S_{PPGMin}; and
wherein the correspondence is defined by the time difference T_{D}, or the phase difference, respectively; and
the operation of generating S 160 the cardiac signal S_{Cardiac} comprises:
   generating an indication of an expected time R_{PeakEst}, or an expected phase, of a reference point R_{Peak}, QRS_{End}, T_{End} in a cardiac cycle T_{HB} of the heart, based on the trigger point S_{PPGMax}, S_{PPGMin} in the subsequent surrogate cardiac signal S_{PPG}', and the time difference T_{D}, or the phase difference, and a known time or phase relationship ΔT_{RP} between the reference cardiac signal S_{Image} and the reference point R_{Peak}, QRS_{End}, T_{End} in the cardiac cycle T_{HB}.

In this example, the operation of establishing a trigger point S_{PPGMax}, S_{PPGMin} in the subsequent surrogate cardiac signal S_{PPG}' may be performed using known signal processing techniques. As described above, various trigger points may be used. For instance, the trigger point may correspond to a maximum value S_{PPGMax} of the subsequent surrogate cardiac signal S_{PPG}', or a minimum value S_{PPGMin} of the subsequent surrogate cardiac signal S_{PPG}', or a maximum value of a derivative of the subsequent surrogate cardiac signal S_{PPG}', or a minimum value of a derivative of the subsequent surrogate cardiac signal S_{PPG}', or a zero crossing value of a Hilbert transform of the subsequent surrogate cardiac signal S_{PPG}'.

In this example, the correspondence is then defined by a time difference, or a phase difference, between a reference point S_{ImageMax} in the reference cardiac signal S_{Image} and the trigger point S_{PPGMax}, S_{PPGMin}. The reference point R_{Peak} may be the R-peak, or the end of the QRS complex QRS_{End}, or the end of the T-wave T_{End}, for example.

In this example, the operation of generating an indication of an expected time R_{PeakEst}, or an expected phase, of a reference point R_{Peak}, QRS_{End}, T_{End} in a cardiac cycle T_{HB} of the heart, is performed in the same manner as described above, with the correspondence being evaluated as the time difference T_{D}, or the phase difference.

Referring now to the operation S170 illustrated in Fig. 2; in this operation, the cardiac signal S_{Cardiac}, is outputted. The cardiac signal S_{Cardiac} may be outputted in various ways, including to a display device such as to the display 160 illustrated in Fig. 1, or to a virtual/ augmented reality display device, or to a printer, or to a computer-readable storage medium, or to the internet, or to the Cloud, and so forth. The cardiac signal S_{Cardiac} may be used in various applications. For instance, the cardiac signal S_{Cardiac} may be used to assess the health of a subject. In this situation, the cardiac signal S_{Cardiac} may simply be outputted. For instance, the cardiac signal S_{Cardiac} may be outputted to a display device, e.g. in the form of a graph, in order for a physician to assess the heart rate, or a heart rate variability of a subject. In other applications, the cardiac signal S_{Cardiac} may be used for other purposes, such as to control the acquisition of medical image data, or to select medical image data for reconstruction into a medical image of the subject. In such applications, the cardiac signal S_{Cardiac} may be outputted to a medical imaging system, or to a radiation therapy system, or to a computer readable storage medium. These applications are described in more detail below.

It is noted that the system 100 may also include one or more of: a medical imaging system for providing the medical image data 120, such as for example the MRI system 150 illustrated in Fig. 1; one or more sensors 140a, 140b, such as for example a PPG, sensor 140a, 140b, or a BCG sensor, or a pulse oximetry sensor, or a pressure sensor, or a radar sensor; a display, such as the display 160 illustrated in Fig. 1, for displaying the cardiac signal S_{Cardiac}, and other outputs generated by the one or more processors 110; a patient bed 170; and a user input device (not illustrated in Fig. 1) configured to receive user input in relation to the operations performed by the one or more processors 110, such as a keyboard, a mouse, a touchscreen, and so forth.

In another example, a computer-implemented method of providing a cardiac signal S_{Cardiac}, is provided. The method comprises:
receiving S110 medical image data 120 representing a beating of a heart of a subject;
receiving S120 initial surrogate cardiac signal data 130 comprising an initial surrogate cardiac signal S_{PPG} representing the beating of the heart of the subject, the initial surrogate cardiac signal data 130 being generated contemporaneously with the medical image data;
analyzing S130 the medical image data 120 to provide a reference cardiac signal S_{Image} representing the beating of the heart of the subject;
determining S140 a correspondence T_{D} between the reference cardiac signal S_{Image} and the initial surrogate cardiac signal S_{PPG};
receiving S150 subsequent surrogate cardiac signal data 130' comprising a subsequent surrogate cardiac signal S_{PPG}' representing the beating of the heart of the subject;
generating S160 the cardiac signal S_{Cardiac} based on the subsequent surrogate cardiac signal S_{PPG}' and the correspondence T_{D}; and
outputting S170 the cardiac signal S_{Cardiac}.

In another example, a computer program product is provided. The computer program product comprises instructions which when executed by one or more processors 110, cause the one or more processors to carry out a method of providing a cardiac signal S_{Cardiac}. The method comprises:
receiving S110 medical image data 120 representing a beating of a heart of a subject;
receiving S120 initial surrogate cardiac signal data 130 comprising an initial surrogate cardiac signal S_{PPG} representing the beating of the heart of the subject, the initial surrogate cardiac signal data 130 being generated contemporaneously with the medical image data;
analyzing S130 the medical image data 120 to provide a reference cardiac signal S_{Image} representing the beating of the heart of the subject;
determining S140 a correspondence T_{D} between the reference cardiac signal S_{Image} and the initial surrogate cardiac signal S_{PPG};
receiving S150 subsequent surrogate cardiac signal data 130' comprising a subsequent surrogate cardiac signal S_{PPG}' representing the beating of the heart of the subject;
generating S160 the cardiac signal S_{Cardiac} based on the subsequent surrogate cardiac signal S_{PPG}' and the correspondence T_{D}; and
outputting S170 the cardiac signal S_{Cardiac}.

As mentioned above, the system 100 illustrated in Fig. 1 may be used in various cardiac gating applications. In one example, the system 100 is used to perform cardiac gating for the purpose of reconstructing medical images. In this example, a medical imaging system 150 is provided. The medical imaging system 150 comprises:
the system 100;
one or more sensors 140a, 140b configured to generate the surrogate cardiac signal data S_{PPG}, S_{PPG}'; and
one or more processors;
wherein the medical imaging system 150 is configured to acquire the medical image data 120, and to provide the medical image data to the one or more processors 110 of the system 100; and
wherein the one or more processors of the medical imaging system 150 are configured to:
   receive the cardiac signal S_{Cardiac}; and
   trigger the acquisition of subsequent medical image data by the medical imaging system 150 based on the received cardiac signal S_{Cardiac};
      or
   cause the medical imaging system 150 to acquire subsequent medical image data; and
   timestamp the acquired subsequent medical image data based on the received cardiac signal S_{Cardiac}.

The first of the options described above may be referred-to as prospective gating. The second of the options described above may be referred-to as retrospective gating. As described above, the use of either gating technique may be used to provide medical image data in which the anatomy is in a similar position, and thereby reduce the impact of cardiac motion-induced blurring in images that are subsequently reconstructed from the data. In this example, the medical imaging system 150 may be any of the medical imaging systems mentioned above. For instance it may be a (spectral) X-ray projection imaging system, a 2D/ 3D ultrasound imaging system, an MRI system, a (spectral) CT imaging system, a PET imaging system, or a SPECT imaging system, and so forth. The one or more sensors 140a, 140b may be provided by various sensors, including for example a photoplethysmography, PPG, sensor 140a, 140b, or a ballistocardiographic, BCG, sensor, or a pulse oximetry sensor, or a pressure sensor, or a radar sensor. In the case of the medical imaging system being a CT system, the gating may be performed during so-called step-and-shoot imaging procedures, or in a helical imaging procedure. In either case, prospectively gating the acquisition of the subsequent medical image data may include using the cardiac signal S_{Cardiac} to modulate the X-ray dose.

The subsequent medical image data in this example is medical image data that is acquired by the medical imaging system 150 at a later point in time to the acquired medical image data 120. In one example, the medical image data 120 represents a surview scan of the subject, or scan of an injected contrast agent bolus in the subject, and the subsequent medical image data represents a diagnostic imaging scan of the subject. As mentioned above, a surview scan, and a scan of an injected contrast agent bolus in the subject, are typically acquired as part of the conventional workflow during medical imaging procedures, and they typically include sufficient information to represent the beating of a heart of a subject. Thus, the use of such scans to provide the medical image data 120 simplifies workflow and/or reduces X-ray dose to a subject.

In a related example, the medical image data that is acquired is reconstructed. In this example, the one or more processors of the medical imaging system are configured to:
reconstruct one or more medical images from the subsequent medical image data;
   or
select medical image data from the subsequent medical image data based on the timestamping; and
reconstruct the selected medical image data to provide one or more medical images, respectively.

As described above, the effect of this is to provide reconstructed medical images with reduced cardiac motion-induced blurring..

In another example, the system 100 is used to perform cardiac gating for the purpose of triggering the deliver of radiation therapy to a subject..

In this example, a radiation therapy delivery system, is provided. The radiation therapy delivery system comprises:
the system 100;
a medical imaging system 150; and
one or more sensors 140a, 140b configured to generate the surrogate cardiac signal data S_{PPG}, S_{PPG}'; and
one or more processors;
wherein the medical imaging system 150 is configured to acquire the medical image data 120, and to provide the medical image data to the one or more processors 110 of the system 100; and
wherein the one or more processors of the radiation therapy delivery system are configured to:
   receive the cardiac signal S_{Cardiac}; and
   trigger the delivery of radiation to a subject based on the received cardiac signal S_{Cardiac}.

In this example, the radiation therapy delivery system may be an external beam radiation therapy delivery system. The beam may be a proton beam, an electron beam, or an X-ray beam. The delivery of therapeutic radiation using an X-ray beam is typically referred-to as radiotherapy. In this example, the intensity of the beam of radiation may be modulated based on the received cardiac signal S_{Cardiac}. For example radiation may be delivered to the subject only in periods corresponding to a known point in the cardiac cycle. In so doing, the heart, and consequently the anatomy of the subject, is in a known position in the periods in which the radiation is delivered to the anatomy. In other words, it reduces the impact of cardiac motion. This helps to reduce the amount of healthy tissue that is affected by the radiation.

The above examples are to be understood as illustrative of the present disclosure, and not restrictive. Further examples are also contemplated. For instance, the examples described in relation to the system 100, may also be provided by the computer-implemented method, or by the computer program product, or by the computer-readable storage medium, or by the medical imaging system 150, or by the radiation therapy delivery system, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for providing a cardiac signal (S_{Cardiac}), the system comprising one or more processors (110) configured to:
receive (S110) medical image data (120) representing a beating of a heart of a subject;
receive (S120) initial surrogate cardiac signal data (130) comprising an initial surrogate cardiac signal (S_{PPG}) representing the beating of the heart of the subject, the initial surrogate cardiac signal data (130) being generated contemporaneously with the medical image data (120);
analyze (S130) the medical image data (120) to provide a reference cardiac signal (S_{Image}) representing the beating of the heart of the subject;
determine (S140) a correspondence (T_{D}) between the reference cardiac signal (S_{Image}) and the initial surrogate cardiac signal (S_{PPG});
receive (S150) subsequent surrogate cardiac signal data (130') comprising a subsequent surrogate cardiac signal (S_{PPG}') representing the beating of the heart of the subject;
generate (S 160) the cardiac signal (S_{Cardiac}) based on the subsequent surrogate cardiac signal (S_{PPG}') and the correspondence (T_{D}); and
output (S170) the cardiac signal (S_{Cardiac}).

2. The system according to claim 1, wherein the generating (S160) the cardiac signal (S_{Cardiac}) comprises generating an indication of an expected time (R_{PeakEst}), or an expected phase, of a reference point (R_{Peak}, QRS_{End}, T_{End}) in a cardiac cycle (T_{HB}) of the heart, and wherein the indication is generated based on the subsequent surrogate cardiac signal (S_{PPG}'), and the correspondence (T_{D}), and based further on a known time or phase relationship (ΔT_{RP}) between the reference cardiac signal (S_{Image}) and the reference point (R_{Peak}, QRS_{End}, T_{End}) in the cardiac cycle (T_{HB}).

3. The system according to claim 1, wherein the determining (S140) a correspondence (T_{D}) between the reference cardiac signal (S_{Image}) and the initial surrogate cardiac signal (S_{PPG}), comprises:
establishing a trigger point (S_{PPGMax}, S_{PPGMin}) in the subsequent surrogate cardiac signal (S_{PPG}'); and
calculating a time difference, or a phase difference, between a reference point (S_{ImageMax}) in the reference cardiac signal (S_{Image}) and the trigger point (S_{PPGMax}, S_{PPGMin}); and
wherein the correspondence is defined by the time difference (T_{D}), or the phase difference, respectively; and
wherein the generating (S160) the cardiac signal (S_{Cardiac}) comprises:
generating an indication of an expected time (R_{PeakEst}), or an expected phase, of a reference point (R_{Peak}, QRS_{End}, T_{End}) in a cardiac cycle (T_{HB}) of the heart, based on the trigger point (S_{PPGMax}, S_{PPGMin}) in the subsequent surrogate cardiac signal (S_{PPG}'), and the time difference (T_{D}), or the phase difference, and a known time or phase relationship (ΔT_{RP}) between the reference cardiac signal (S_{Image}) and the reference point (R_{Peak}, QRS_{End}, T_{End}) in the cardiac cycle (T_{HB}).

4. The system according to claim 2 or claim 3, wherein the reference point (R_{Peak}) is the R-peak, or the end of the QRS complex (QRS_{End}), or the end of the T-wave (T_{End}).

5. The system according to claim 3 or claim 4, wherein the trigger point corresponds to a maximum value (S_{PPGMax}) of the subsequent surrogate cardiac signal (S_{PPG}'), or a minimum value (S_{PPGMin}) of the subsequent surrogate cardiac signal (S_{PPG}'), or a maximum value of a derivative of the subsequent surrogate cardiac signal (S_{PPG}'), or a minimum value of a derivative of the subsequent surrogate cardiac signal (S_{PPG}'), or a zero crossing value of a Hilbert transform of the subsequent surrogate cardiac signal (S_{PPG}').

6. The system according to any previous claim, wherein the medical image data (120) comprises a temporal sequence of images including at least a portion of the heart and/or at least a portion of the aorta, and wherein the analyzing (S130) the medical image data (120) comprises:
reconstructing at least a portion of the medical image data (120) to provide a temporal sequence of images representing the at least a portion of the heart and/or the at least a portion of the aorta; and
extracting the reference cardiac signal (S_{Image}) representing the beating of the heart of the subject, from the temporal sequence of images.

7. The system according to claim 6, wherein the extracting the reference cardiac signal (S_{Image}) comprises:
evaluating an image intensity within a region of interest in the images; and
generating the reference cardiac signal (S_{Image}) based on the evaluated image intensities in the images.

8. The system according to claim 7, wherein the region of interest corresponds to a cardiac chamber.

9. The system according to any previous claim, wherein the surrogate cardiac signal data (130, 130') is generated by a photoplethysmography, PPG, sensor (140a, 140b), or by a ballistocardiographic, BCG, sensor, or by a pulse oximetry sensor, or by a pressure sensor, or by a radar sensor.

10. The system according to any previous claim, wherein the medical image data (120) is generated by a computed tomography, CT, imaging system, or by a projection X-ray imaging system, or by a magnetic resonance imaging, MRI, system (150), or by a positron emission tomography, PET, imaging system.

11. The system according to any previous claim, wherein the medical image data (120) represents a surview scan of the subject, or scan of an injected contrast agent bolus in the subject.

12. A computer-implemented method of providing a cardiac signal (S_{Cardiac}), the method comprising:
receiving (S110) medical image data (120) representing a beating of a heart of a subject;
receiving (S120) initial surrogate cardiac signal data (130) comprising an initial surrogate cardiac signal (S_{PPG}) representing the beating of the heart of the subject, the initial surrogate cardiac signal data (130) being generated contemporaneously with the medical image data;
analyzing (S130) the medical image data (120) to provide a reference cardiac signal (S_{Image}) representing the beating of the heart of the subject;
determining (S140) a correspondence (T_{D}) between the reference cardiac signal (S_{Image}) and the initial surrogate cardiac signal (S_{PPG});
receiving (S150) subsequent surrogate cardiac signal data (130') comprising a subsequent surrogate cardiac signal (S_{PPG}') representing the beating of the heart of the subject;
generating (S160) the cardiac signal (S_{Cardiac}) based on the subsequent surrogate cardiac signal (S_{PPG}') and the correspondence (T_{D}); and
outputting (S170) the cardiac signal (S_{Cardiac}).

13. A computer program product comprising instructions which when executed by one or more processors (110), cause the one or more processors to carry out the method according to claim 12.

14. A medical imaging system (150) comprising:
the system (100) according to any one of claims 1 - 12;
one or more sensors (140a, 140b) configured to generate the surrogate cardiac signal data (S_{PPG}, S_{PPG}'); and
one or more processors;
wherein the medical imaging system (150) is configured to acquire the medical image data (120), and to provide the medical image data to the one or more processors (110) of the system (100); and
wherein the one or more processors of the medical imaging system (150) are configured to:
receive the cardiac signal (S_{Cardiac}); and
trigger the acquisition of subsequent medical image data by the medical imaging system (150) based on the received cardiac signal (S_{Cardiac});
or
cause the medical imaging system (150) to acquire subsequent medical image data; and
timestamp the acquired subsequent medical image data based on the received cardiac signal (S_{Cardiac}).

15. The medical imaging system (150) according to claim 14, wherein the one or more processors of the medical imaging system are further configured to:
reconstruct one or more medical images from the subsequent medical image data;
or
select medical image data from the subsequent medical image data based on the timestamping; and
reconstruct the selected medical image data to provide one or more medical images, respectively.
